(19) Europäisches Patentamt
European Patent Office
Office européen des brevets

(11) Veröffentlichungsnummer : **0 068 425**
**B1**

(12) **EUROPÄISCHE PATENTSCHRIFT**

(45) Veröffentlichungstag der Patentschrift : **30.10.85**

(21) Anmeldenummer : **82105500.1**

(22) Anmeldetag : **23.06.82**

(51) Int. Cl.⁴ : **C 07 D293/10**, C 07 C163/00, C 07 B 59/00, A 61 K 43/00, A 61 K 49/02

# (54) Verfahren zur Herstellung von Selen-Methylenblau, radioaktives Selen-Methylenblau, seine Herstellung und Verwendung in Präparaten zur Darstellung von Epithelkörperchenadenomen.

(30) Priorität : **27.06.81 DE 3125296**

(43) Veröffentlichungstag der Anmeldung : **05.01.83 Patentblatt 83/01**

(45) Bekanntmachung des Hinweises auf die Patenterteilung : **30.10.85 Patentblatt 85/44**

(84) Benannte Vertragsstaaten :
**AT BE CH DE FR GB IT LI NL SE**

(56) Entgegenhaltungen :
**US-A- 3 965 049**
**JOURNAL OF MEDICINAL CHEMISTRY, Band 17, Heft 8, 1974, England, J.T. GROVES et al. "Synthesis of Seleno-Toluidine Blue", Seiten 902 bis 904**
**ACTA RADIOL. THER. PHYS. BIOL., Band 11, Heft 4, 1972, T.G. BRIEN "Selenium Labelled Toluidine Blue as an Agent for Parathyroid Scanning", Seiten 312 bis 320**

(73) Patentinhaber : **HOECHST AKTIENGESELLSCHAFT**
**Postfach 80 03 20**
**D-6230 Frankfurt am Main 80 (DE)**

(72) Erfinder : **Waschulzik, Günther, Dr.**
**Bremer Strasse 29**
**D-2740 Bremervörde (DE)**
Erfinder : **Oelschläger, Herbert, Prof. Dr.**
**Georg-Voigt-Strasse 14**
**D-6000 Frankfurt am Main (DE)**
Erfinder : **Schuldes, Helmut**
**Darmstädter Strasse 33**
**D-6080 Gross-Gerau (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 068 425 B1

**Beschreibung**

Selen-Methylenblau, 3,7-Bis-(dimethylamino)-phenoselenazin-5-ium-chlorid, ist nach P. Karrer, Ber. Dtsch. Chem. Ges., *51,* 190-192 (1918) bekannt. Danach entsteht es, wenn man Selenodiphenylamin gleichzeitig oxidiert und bromiert. Das angefallene 3,7-Dibrom-phenoselenaziniumbromid geht mit Dimethylamin in Selen-Methylenblau über.

Die Methode von Karrer ergibt Ausbeuten von etwa 5 % d. Th. Zn Se-Methylenblau. Die Darstellung des als Ausgangsmaterial verwendeten Phenoselenazins durch Umsetzung von Diselendichlorid und Diphenylamin verläuft nach Cornelius W., J. Prakt. Chem., *88,* 395 (1913) wegen der Photoinstabilität des Produktes mit einer Ausbeute von 40 % d. Th., wenn aufgrund neuerer Erfahrungen Chloroform statt Benzol als Lösungsmittel verwendet wird [Podolesov, B.D., V.B. Jordanorska, Croat. Chem. Acta, *42,* 61 (1970)].

Es wurde nun ein Verfahren zur Herstellung von Selen-Methylenblau gefunden, das dadurch gekennzeichnet ist, daß man 3-Brom-N,N-dimethylanilin (I) in einem wasserfreien Lösungsmittel mit Magnesium zu 3-Dimethylaminophenyl-magnesiumbromid (II) umsetzt, durch Zugabe von Selen 3-Dimethylaminophenyl-selenomagnesiumbromid (III) herstellt, dieses durch wäßrige Mineralsäure zu 3-Dimethylamino-selenophenol (IV) hydrolysiert, dieses durch Oxydation in Bis-(3-dimethylaminophenyl)-diselenid (V) überführt, und dies mit 4-Nitroso-N,N-dimethylanilin (VI) oder einem seiner Salze in wäßrigem Lösungsmittel umsetzt.

In dieser Reaktionsfolge wird das Dreiringsystem erst in der letzten Stufe gebildet. Dadurch werden bei Markierung mit $^{75}$Se, dessen Einführung in der zweiten Stufe erfolgt, Verluste vermieden. Die Reaktion wird durch folgendes Formelschema wiedergegeben :

Formelschema :

(I) (II)

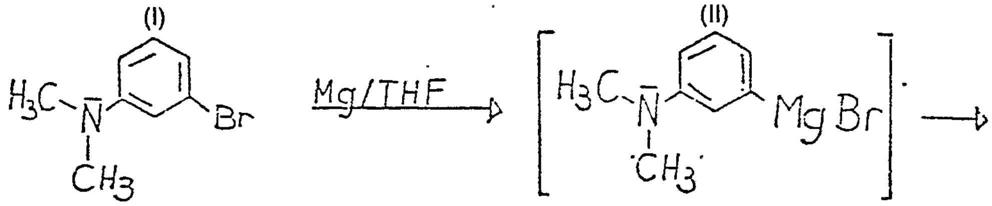


3-Brom-N,N-dimethylanilin $n_D^{20}$ 1,6018 3-Dimethylaminophenylmagnesiumbromid

(III) (IV)

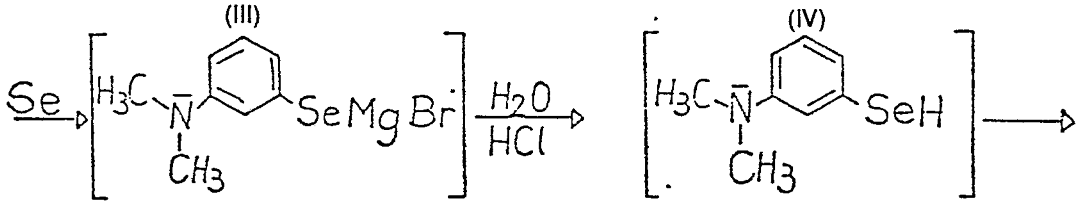


3-Dimethylamino-selenophenol

(V)

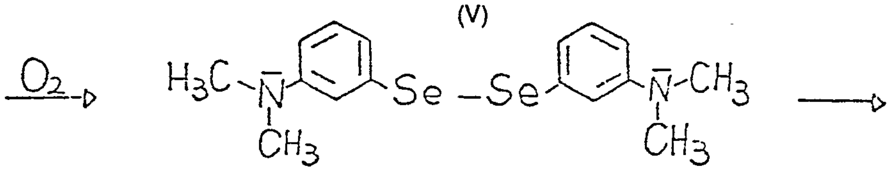


Bis-(3-dimethylaminophenyl)-diselenid (MG 398,27) Schmp. 57-58 °C

(VI) (VII)

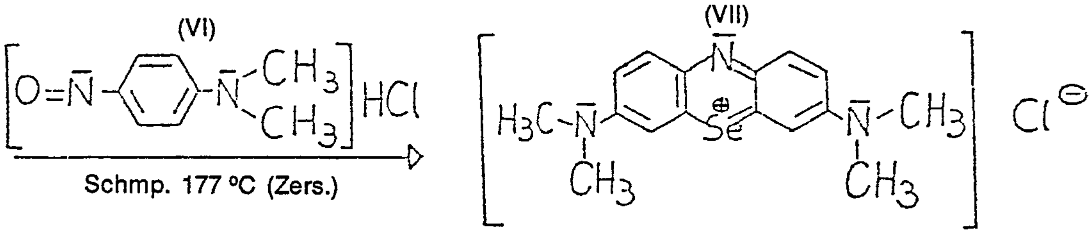


3,7-Bis-(dimethylamino)-phenoselenazin-5-ium-chlorid (MG 366,75) ; Dihydrat (MG 402,79)

Das als Zwischenprodukt isolierbare Bis-(3-dimethylaminophenyl)-diselenid (V) ist bisher nicht beschrieben.

Die erste Stufe des Verfahrens ist eine stark temperaturabhängige Grignard-Reaktion, die über die nicht isolierten Zwischenprodukte (II), (III) und (IV) zu dem Diselenid (V) führt. Es ist vorteilhaft, anstelle von Diethylether einen höher siedenden Ether, vorzugsweise Tetrahydrofuran (THF) zu verwenden. Die Reaktion wird unter Luftausschluß und bevorzugt bei 35-40 °C durchgeführt. Das so erhaltene 3-Dimethylaminophenyl-magnesiumbromid (II) wird bei gleichbleibender Temperatur mit Selen, gegebenenfalls radioaktivem Selen-75, weiterhin unter Luftausschluß zur Selenomagnesium-bromid-Verbindung (III) umgesetzt. Diese Verbindung wird zweckmäßig mit kaltem Wasser und einer Mineralsäure, vorzugsweise Salzsäure, hydrolysiert. Der dabei als Nebenprodukt entstehende, gegebenenfalls radioaktive Selenwasserstoff, wird zweckmäßig in konzentrierter Kalilauge aufgefangen.

Es ist zweckmäßig, die Zwischenstufen (II), (III) und (IV) nicht zu isolieren, sondern das 3-Dimethylamino-selenophenol (IV) in situ zu Bis-(3-dimethylaminophenyl)-diselenid (V) zu oxydieren. Dazu wird zweckmäßig Sauerstoff oder Luft in die Lösung von (IV) geleitet. Auch Wasserstoffperoxid oder Persäuren wie Peroxybenzoesäure sind verwendbar. Das Diselenid (V) wird zweckmäßig durch Extrahieren mit einem geeigneten Lösungsmittel wie Diethylether, Chloroform oder Benzol isoliert, das Lösungsmittel getrocknet, abgedampft und der Rückstand zweckmäßig aus einer Mischung von Alkohol und Wasser, vorzugsweise Ethanol und Wasser, umkristallisiert.

Die anschließende Umsetzung des Diselenids (V) mit 4-Nitroso-N,N-dimethylanilin (VI) oder einem seiner Salze, vorzugsweise dem Hydrochlorid, erfolgt in alkoholisch wäßriger Lösung, vorzugsweise in 60-70 %igem Ethanol, wobei nach langsamer Vereinigung der Reaktionspartner die Reaktionsmischung 8-12 Stunden lang unter Rückfluß erhitzt wird. Danach wird das Lösungsmittel abgedampft, der Rückstand in eine wäßrige Lösung übergeführt und daraus zweckmäßig durch Aussalzen isoliert.

Der Schmelzpunkt für das dunkelgrüne kristalline Pulver ist mit Hilfe der Kapillarmethode schwer zu ermitteln. Dagegen läßt sich aus dem Schmelzdiagramm (Fus-O-mat(R) Heraeus) recht genau ein Schmelzintervall von 173-175 °C (Z) festlegen.

Strukturbeweisend sind auch das IR-Sprektrum und das Massenspektrum der Substanz. Der basepeak im Massenspektrum ist der $M^+ + 1$-Peak (m/e 333), er korrespondiert mit der Leukoform.

| Elementaranalyse : (Dihydrat) | Ber.% | Gef.% |
|---|---|---|
| | C 47,7 | C 47,1 |
| | H 5,5 | H 5,52 |
| | Cl 8,8 | Cl 9,4 |
| | N 10,4 | N 10,5 |
| | O 8,0 | O 8,4 |
| | Se 19,6 | Se 19,8 |

Bisher bekannte Syntheseverfahren für Selen-Methylenblau eigneten sich nicht nur wegen sehr geringer Ausbeuten und ungenügender chemischer Reinheit des Endprodukts schlecht zur Gewinnung von $^{75}$Se-markiertem Selen-Methylenblau, sondern in erster Linie wegen der Notwendigkeit, das Selen-75 über komplizierte, zusätzliche Synthesestufen, z. B. als $Se_2Cl_2$ bzw. KSeCN, einzuführen. Der größere Vorteil des Verfahrens gemäß der Erfindung besteht darin, daß man direkt elementares Selen- und als solches liegt auch Selen-75 vor, das im Reaktor durch eine n, γ-Reaktion aus Selen-74 gewonnen wird — einsetzt, und außerdem in der relativ guten Ausbeute des Selen-Methylenblaus, bezogen auf das eingesetzte Selen.

Klinische Untersuchungen, die mit der nicht radioaktiv markierten Substanz durchgeführt wurden [G. Waschulzik, Therapiewoche, 28, 3697 (1978) : « Chirurgische Erfahrungen über 117 Fälle mit Hyperparathyreoidismus »] zeigten, daß durch Anfärbung mit dem Vitalfarbstoff Methylenblau die Sichtbarmachung der Nebenschilddrüsen möglich ist, wodurch die Lokalisierung von Epithelkörperchenadenomen und deren erfolgreiche chirurgische Entfernung ermöglicht wird. Das Anreicherungsverhältnis für Nebenschilddrüse zu normalem Gewebe betrug etwa 10 : 1. Das Auffinden der Adenome nach Anfärbung mit dem nicht radioaktiven Vitalfarbstoff ist aber nur durch einen chirurgischen Eingriff möglich.

Ein Auffinden vor einem chirurgischen Eingriff setzt radioaktive Markierung der Substanz mit einem γ-Strahler voraus, dessen Strahlung mit einem geeigneten Detektor außerhalb des Körpers der untersuchten Person nachweisbar ist. Das ist ohne chemische Veränderung der Substanz durch Substitution des Selens durch Selen-75 in Selen-Methylenblau möglich.

Radioaktiv markiertes $^{75}$Selen-Methylenblau eignet sich daher zum Auffinden und zur topographischen Darstellung von Epithelkörperchenadenomen. Zu diesem Zweck kann es in blutisotonischer Lösung in einer Dosis von 200 bis 800, vorzugsweise 300 bis 500 μCi pro Anwendung, und zwar vorzugsweise in die Vena cubiti injiziert oder als Tropfinfusion appliziert werden.

## Herstellungsbeispiele

Die Ausgangssubstanz 3-Brom-N,N-dimethylanilin (I) wurde nach Gilman und Banner[1)] synthetisiert, aus 3-Bromanilin und 3 Äquivalenten Dimethylsulfat in wäßriger Lösung, und Extraktion von (I) mit Ether.

1) H. Gilman, J. Banner, J. Amer. Chem. Soc., *62*, 344-345 (1940).

1. Darstellung von Bis-(3-dimethylaminophenyl)-diselenid (V)

1.1 3-Dimethylamino-selenophenol (IV)

Das Grignard-Reagenz (II) wurde aus 1,22 g (0,05 Mol) Mg-Spänen und 10 g (0,05 Mol) (I) in 40 ml wasserfreiem THF hergestellt. Zunächst werden die Mg-Späne in 15 ml THF vorgelegt; Luftsauerstoff wird mittels $N_2$ vertrieben. Unter Rühren wird bis zum Anspringen der Reaktion reines (I) (ca. 2 g) zugetropft, gegebenenfalls wird bis auf 30 °C erwärmt. Nach Einsetzen der Reaktion wird restliches (I) — gelöst in 25 ml THF — in der Weise zugetropft, daß die Temperatur 40 °C nicht übersteigt, gegebenenfalls wird mit Eis gekühlt. Nach beendeter Zugabe wird noch 30 min. bei ca. 35 °C gerührt.

Danach werden 3,95 g (0,05 Mol) Selen plv., schwarz, (24 h über $H_2SO_4$ conc. getrocknet) so eingebracht, daß die Temperatur des Reaktionsansatzes ohne Erwärmen zwischen 35 und 40 °C gehalten wird. Nach beendeter Zugabe wird noch ca. 30 min. gerührt. Es entsteht die Selenomagnesiumbromid-Verbindung (III), die ohne Isolierung weiter verarbeitet wird.

Hierzu werden ca. 60 g gemahlenes Eis, anschließend 13 ml HCl 20 % zugesetzt. Bei der Hydrolyse entstandener toxischer $H_2Se$ wird zur Entgiftung in KOH 50 % geleitet. Ohne das Selenophenol (IV) zu isolieren, wird die Oxidation zu (V) wie folgt durchgeführt:

1.2 Bis-(3-dimethylaminophenyl)-diselenid (V)

Der Reaktionsansatz wird 30 min. mit $O_2$ oder mit Luft begast. Zur Isolierung des entstandenen Diselenids (V) wird mit $Na_2CO_3$ auf pH 8-9 eingestellt, mit Ether extrahiert und über $Na_2SO_4$ getrocknet. Das nach Abdestillieren des Ethers anfallende Öl kristallisiert orange aus. Durch Umkristallisieren aus Ethanol/Wasser erhält man intensiv gelb gefärbte Plättchen.

Schmp. 57-58 °C; Ausbeute: 5,4 g (54,2 % d. Th.).

2. 3,7-Bis-(dimethylamino)-phenoselenazin-5-ium-chlorid (VII)

1,0 g (0,0025 Mol) (V) wird in 70 ml Ethanol 96 % gelöst und zum Sieden erhitzt, 0,94 g (0,005 Mol) 4-Nitroso-N,N-dimethylanilin-HCl (VI) werden, gelöst in 30 ml Wasser, innerhalb 1 h zugetropft und der Ansatz 10 h unter Rückfluß erwärmt. Danach destilliert man das Lösungsmittel ab, der Rückstand wird zur Extraktion des wasserlöslichen Anteils mit wenig Wasser digeriert und filtriert. Die erhaltene hochkonzentrierte saure Lösung wird zum Aussalzen mit dem gleichen Volumen einer gesättigten NaCl-Lösung versetzt. Das ausgefallene Produkt (VII) wird zur Reinigung mehrmals aus Ethanol/Wasser umkristallisiert, bis das dünnschichtchromatographische Verhalten ($CHCl_3/CH_3OH/NH_3$ conc. 80/20/1, Kieselgel 60 $F_{254}$ Merck, Art.-Nr. 5720) dem von Methylenblau DAB 7 entspricht. Man erhält ein dunkelgrünes, metallisch glänzendes, kristallines Pulver, das ein Schmelzintervall von 173-175 °C zeigt (Fus-O-mat[R], Heraeus).

Ausbeute: 0,74 g (40,5 % d. Th.)

3. Darstellung von $^{75}$Se-Methylenblau

Das als Ausgangssubstanz benötigte 3-Brom-N,N-dimethylanilin wird nach (1) durch Umsetzung von m-Bromanilin mit Dimethylsulfat hergestellt. Bei einem 0,5 Mol-Ansatz erhält man die Substanz in rund 50 % Ausbeute.

Sdp. = 118 – 119 °C/8 mmHg.

3.1 Aus 0,305 g Mg-Spänen und 2,5 g 3-Brom-N,N-dimethylanilin (I) wird in 15 ml wasserfreiem Tetrahydrofuran zuerst die Grignardverbindung (II) hergestellt. Dazu legt man unter Sauerstoffausschluß die Mg-Späne in 5 ml THF vor und tropft (I), gelöst in 10 ml THF zu, wobei die Temperatur 40 °C nicht überschreiten darf. Anschließend rührt man weitere 30 min. bei ca. 35 °C.

Danach werden 0,99 g Selen-75 (50 mCi) unter weiterem Luftausschluß langsam zugesetzt, so daß die Temperatur des Reaktionsansatzes ohne Erwärmen zwischen 35 und 40 °C gehalten werden kann. Man rührt nach beendeter Zugabe noch 30 min. Zu der so erhaltenen Selenomagnesiumbromidverbindung (III) setzt man vorsichtig ca. 15 ml kaltes Wasser und anschließend 3,2 ml 20 %ige Salzsäure zu. Bei der Hydrolyse als Nebenprodukt entstehender Selenwasserstoff-$^{75}$Se wird in 50 %iger KOH aufgefangen.

3.2 Ohne Isolierung des so erhaltenen 3-Dimethylamino-selenophenols (IV) wird durch 30-minütiges Begasen der Reaktionslösung mit Sauerstoff die Oxidation zum Diselenid (V) erreicht. Man stellt mit $Na_2CO_3$ auf pH 8-9 ein, extrahiert mit Ether und trocknet den Extrakt über $Na_2SO_4$. Nach dem Abdestillieren des Ethers fällt der vorher ölige Rückstand als orange gefärbte kristalline, radiochemisch reine Masse an.

Ausbeute: 1,0 g = 40,0 %, bezogen auf Selen.

1,0 g (2,5 mMol) (V) löst man in 70 ml 96 %igem Ethanol und erhitzt zum Sieden. Man tropft 30 ml einer wäßrigen Lösung von 0,94 g (5 mMol) 4-Nitroso-N,N-dimethylanilinhydrochlorid (VI) innerhalb von 1 h zu und erwärmt 10 h unter Rückfluß. Ethanol wird abdestilliert und der Rückstand zur Extraktion des wasserlöslichen Anteils mit wenig Wasser digeriert und filtriert. Die konzentrierte saure Lösung wird mit dem gleichen Volumen einer gesättigten Natriumchloridlösung versetzt, wobei (VII) ausfällt. Nach der Reinigung durch säulenchromatographische Trennung erhält man ein dunkelgrünes, kristallines Pulver mit einer spezifischen Aktivität von 10,85 mCi/g (3,98 mCi/mMol). Ausbeute : 0,54 g = 29,3 % bzw. bezogen auf Selen 11,7 %. Die Substanz zeigt bei der dünnschichtchromatographischen Prüfung das gleiche Verhalten wie Methylenblau (DAB 7).

**Patentansprüche** (für die Vertragsstaaten : BE, CH, DE, FR, GB, IT, LI, NL, SE)

1. Verfahren zur Herstellung von Selen-Methylenblau, dadurch gekennzeichnet, daß man 3-Brom-N,N-dimethylanilin in einem wasserfreien Lösungsmittel mit Magnesium zu 3-Dimethylaminophenylmagnesiumbromid umsetzt, durch Zugabe von Selen 3-Dimethylaminophenyl-selenomagnesiumbromid herstellt, dieses durch wäßrige Mineralsäure zu 3-Dimethylamino-selenophenol hydrolysiert, dieses durch Oxydation in Bis-(3-dimethylaminophenyl)-diselenid überführt, und dies mit 4-Nitroso-N,N-dimethylanilin oder einem seiner Salze in einem wäßrigen Lösungsmittel umsetzt.
2. Verfahren zur Herstellung von radioaktivem $^{75}$Selen-Methylenblau, dadurch gekennzeichnet, daß man in dem Verfahren nach Anspruch 1 $^{75}$Selen verwendet.
3. $^{75}$Selen-Methylenblau.
4. Präparat zur Abbildung von Epithelkörperchenadenomen, gekennzeichnet durch einen Gehalt an $^{75}$Selen-Methylenblau.
5. Bis-(3-dimethylaminophenyl)-diselenid.

**Patentansprüche** (für den Vertragsstaat AT)

1. Verfahren zur Herstellung von Selen-Methylenblau, dadurch gekennzeichnet, daß man 3-Brom-N,N-dimethylanilin in einem wasserfreien Lösungsmittel mit Magnesium zu 3-Dimethylaminophenylmagnesiumbromid umsetzt, durch Zugabe von Selen 3-Dimethylaminophenyl-selenomagnesiumbromid herstellt, dieses durch wäßrige Mineralsäure zu 3-Dimethylamino-selenophenol hydrolysiert, dieses durch Oxydation in Bis-(3-dimethylaminophenyl)-diselenid überführt, und dies mit 4-Nitroso-N,N-dimethylanilin oder einem seiner Salze in einem wäßrigen Lösungsmittel umsetzt.
2. Verfahren zur Herstellung von radioaktivem $^{75}$Selen-Methylenblau, dadurch gekennzeichnet, daß man in dem Verfahren nach Anspruch 1 $^{75}$Selen verwendet.
3. Präparat zur Abbildung von Epithelkörperchenadenomen, gekennzeichnet durch einen Gehalt an $^{75}$Selen-Methylenblau.

**Claims** (for the Contracting States : BE, CH, DE, FR, GB, IT, LI, NL, SE)

1. A process for the preparation of selenium methylene blue, which comprises reacting 3-bromo-N,N-dimethyl aniline in an anhydrous solvent with magnesium to yield 3-dimethylaminophenyl magnesium bromide, adding selenium to obtain 3-dimethyl-aminophenyl selenomagnesium bromide, hydrolyzing said compound by means of aqueous mineral acid to yield 3-dimethylamino-selenophenol, converting said compound by oxidation into bis-(3-dimethylaminophenyl)-diselenide and reacting said compound with 4-nitroso-N,N-dimethyl aniline or one of its salts in an aqueous solvent.
2. A process for the preparation of radioactive $^{75}$selenium methylene blue, with comprises using in the process of claim 1 $^{75}$selenium.
3. $^{75}$Selenium methylene blue.
4. Preparation for visualizing parathyroid adenomae, characterized by a content of $^{75}$selenium methylene blue.
5. Bis-(3-dimethylaminophenyl)-diselenide.

**Claims** (for the Contracting State AT)

1. A process for the preparation of selenium methylene blue, which comprises reacting 3-bromo-N,N-dimethyl aniline in an anhydrous solvent with magnesium to yield 3-dimethylaminophenyl magnesium bromide, adding selenium to obtain 3-dimethyl-aminophenyl selenomagnesium bromide, hydrolyzing said compound by means of aqueous mineral acid to yield 3-dimethylamino-selenophenol, converting said compound by oxidation into bis-(3-dimethylaminophenyl)-diselenide and reacting said compound with 4-nitroso-N,N-dimethyl aniline or one of its salts in an aqueous solvent.

2. A process for the preparation of radioactive $^{75}$selenium methylene blue, with comprises using in the process of claim 1 $^{75}$selenium.

3. Preparation for visualizing parathyroid adenomae, characterized by a content of $^{75}$selenium methylene blue.

**Revendications** (pour les Etats contractants : BE, CH, DE, FR, GB, IT, LI, NL, SE)

1. Procédé de préparation de bleu de méthylène sélénié, caractérisé en ce que l'on fait réagir la 3-bromo-N,N-diméthylaniline dans un solvant anhydre avec du magnésium pour obtenir le bromure de 3-diméthylaminophényl-magnésium, on prépare le bromure de 3-diméthylaminophényl-sélénomagnésium par addition de sélénium, on hydrolyse par un acide minéral aqueux en 3-diméthylamino-sélénophénol, on convertit ce dernier par oxydation en diséléniure de bis-(3-diméthylaminophényle) qu'on fait réagir avec la 4-nitroso-N,N-diméthylaniline ou un de ses sels dans un solvant aqueux.

2. Procédé pour la préparation de bleu de méthylène sélénié marqué avec du sélénium-75 radioactif, caractérisé en ce que l'on utilise du sélénium-75 dans le procédé selon la revendication 1.

3. Bleu de méthylène sélénié marqué avec du sélénium-75.

4. Préparation pour la visualisation des adénomes de corpuscules épithéliaux, caractérisée par une teneur en bleu de méthylène sélénié marqué au sélénium-75.

5. Diséléniure de bis-(3-diméthylaminophényle).

**Revendications** (pour l'Etat contractant AT)

1. Procédé pour la préparation de bleu de méthylène sélénié, caractérisé en ce qu'on fait réagir la 3-bromo-N,N-diméthylalinine dans un solvant anhydre avec du magnésium pour obtenir le bromure de 3-diméthylaminophényl-magnésium, on prépare le bromure de 3-diméthylaminophényl-sélénomagnésium par addition de sélénium, on hydrolyse en 3-diméthylamino-sélénophénol par un acide minéral aqueux, on convertit par oxydation en diséléniure de bis-3-diméthylaminophényle et on fait réagir ce dernier avec la 4-nitroso-N,N-diméthylaniline ou un de ses sels dans un solvant aqueux.

2. Procédé pour la préparation de bleu de méthylène sélénié marqué au sélénium-75 radioactif, caractérisé en ce qu'on utilise le sélénium-75 dans le procédé selon la revendication 1.

3. Préparation pour la visualisation des adénomes de corpuscules épithéliaux, caractérisée par une teneur en bleu de méthylène sélénié marqué au sélénium-75.